# EUROPÄISCHE PATENTSCHRIFT

(11) **EP 3 159 727 B1**
(45) Veröffentlichungstag und Bekanntmachung des Hinweises auf die Patenterteilung: **10.04.2019**
(21) Anmeldenummer: 16194640.5
(22) Anmeldetag: 19.10.2016
(51) Int. Cl.: G02B 21/00, G02B 21/36, G02B 21/24, G02B 27/00, G02B 21/22, G06T 5/50, A61B 90/00

(54) **ELEKTRONISCHES (OPERATIONS)MIKROSKOP**
ELECTRONIC (SURGICAL) MICROSCOPE
MICROSCOPE (D'OPÉRATION) ELECTRONIQUE

(30) Priorität: 23.10.2015 DE 102015118154
(43) Veröffentlichungstag der Anmeldung: 26.04.2017
(73) Patentinhaber: Arnold&Richter Cine Technik GmbH&Co. Betriebs KG, 80799 München (DE)
(72) Erfinder: Kiening, Hans, 83661 Lenggries (DE); Geissler, Peter, 81379 München (DE)
(74) Vertreter: Manitz Finsterwald Patent- und Rechtsanwaltspartnerschaft mbB

(56) Entgegenhaltungen:
- DE-A1-102005 032 354
- US-A1- 2004 264 765

## Beschreibung

Die vorliegende Erfindung betrifft ein elektronisches Mikroskop, insbesondere ein Operationsmikroskop, mit einer Kameraeinheit, die zumindest einen elektronischen Bildsensor zum Erzeugen von Bilddatensätzen und eine Abbildungsoptik zum Erzeugen eines Abbildes eines Objekts, insbesondere einer Operationsstelle, auf dem Bildsensor umfasst. Zum Anzeigen derjenigen Bilder, welche den erzeugten Bilddatensätzen entsprechen, ist an dem Mikroskop ein elektronischer Sucher vorgesehen.

Solche elektronischen Mikroskope werden beispielsweise von einem Chirurgen dazu verwendet, eine Operation mit den Vorzügen einer durch das Mikroskop bewirkten optischen Vergrößerung der Operationsstelle durchzuführen. Hierbei betrachtet oder überwacht der Chirurg die Operationsstelle zumindest zeitweise lediglich über den elektronischen Sucher (z.B. zwei kleinformatige Monitore mit zwei aufgesetzten Okularen), auf welchem die mit dem oder den Bildsensor(en) erfasste Bildinformation visuell und insbesondere dynamisch in Echtzeit wiedergegeben wird. Zum Einsatz kommen hierbei elektronische Stereomikroskope, wie sie beispielsweise aus DE 10 2012 218 863 A1 und DE 10 2013 208 306 A1 bekannt sind.

Die Anforderungen an den Bildsensor und an die Abbildungsoptik des Mikroskops können insbesondere im medizinischen Einsatzbereich widersprüchlich sein: Einerseits sind ein großer Bildsensor und eine große Apertur der Abbildungsoptik (d.h. Öffnungsweite) wünschenswert, um eine hohe optische Empfindlichkeit des Mikroskops zu erreichen und eine relativ schwache Beleuchtung einsetzen zu können, die das Körpergewebe nicht unnötig erwärmt. Andererseits kann je nach Anwendungsgebiet, und insbesondere für kurze Brennweiten (d.h. im Weitwinkelbereich), eine größere Schärfentiefe erwünscht sein (d.h. ein größerer Tiefenbereich, der scharf abgebildet wird), um Strukturen in unterschiedlichem Abstand von dem Mikroskop deutlich aufzulösen. Diese Anforderung könnte durch eine kleinere Apertur erfüllt werden, die jedoch nicht erwünscht ist, da dann wiederum eine stärkere Beleuchtung erforderlich wäre.

US 2004/0264765 A1 offenbart ein elektronisches Mikroskop mit den Merkmalen des Obergriffs von Anspruch 1.

DE 10 2005 032 354 A1 offenbart eine Vorrichtung zur Bildaufnahme mit erweitertem Tiefenschärfebereich, insbesondere im Zuge einer mikroskopischen Abtastung einer Probe, mit einer Verstelleinheit für eine Optikeinheit, und mit einer Steueranlage, welche die Verstelleinheit und ggf. die Optikeinheit steuert, wobei die Steueranlage einen variierbaren Fokusstellbereich für die Optikeinheit vorgibt und als Folge hiervon mehrere Einzelbilder mit unterschiedlich kontrastreichen Sektionen aufnimmt sowie zu einem Gesamtbild aus den jeweils kontrastreichsten Sektionen der Einzelbilder in Echtzeit erzeugt, und wobei das jeweils aufgenommene Einzelbild zunächst unter Rückgriff auf eine gemessene oder angenommene optische Übertragungsfunktion entfaltet und erst dann hinsichtlich seines Kontrastes ausgewertet wird.

Es ist eine Aufgabe der Erfindung, ein verbessertes elektronisches Mikroskop anzugeben, welches Bilder mit größerer Schärfentiefe erfassen und anzeigen kann, ohne dass hierfür die Apertur der Abbildungsoptik verringert werden muss.

Die Aufgabe wird gelöst durch ein Mikroskop mit den Merkmalen des Anspruchs 1. Das Mikroskop weist (i) eine Stelleinrichtung zum Verändern einer Fokuslage der Kameraeinheit, (ii) eine Steuereinrichtung und (iii) eine Recheneinrichtung auf, wobei die Steuereinrichtung dazu angepasst ist, die Stelleinrichtung zu einem sich zyklisch wiederholenden Verändern der Fokuslage der Kameraeinheit zwischen mehreren Fokuswerten anzusteuern und den oder die Bildsensor(en) zum Erzeugen eines jeweiligen Primär-Bilddatensatzes für jeden der mehreren Fokuswerte anzusteuern. Die Recheneinrichtung ist dazu angepasst, aus den für die mehreren Fokuswerte erzeugten Primär-Bilddatensätzen einen Sekundär-Bilddatensatz zu ermitteln, der relativ zu den jeweiligen Primär-Bilddatensätzen eine erweiterte Schärfentiefe aufweist. Der elektronische Sucher ist dazu angepasst, ein dem Sekundär-Bilddatensatz entsprechendes Bild anzuzeigen. Ferner ist das elektronische Mikroskop als ein Stereomikroskop ausgebildet, wobei die Steuereinrichtung dazu angepasst ist, für jeden der mehreren Fokuswerte den zumindest einen Bildsensor zum Erzeugen eines jeweiligen Primär-Bilddatensatzes eines Rechts-Kanals und eines Links-Kanals anzusteuern. Generell können die stereoskopischen Bilddatensätze entweder mittels eines einzigen Bildsensors erzeugt werden, insbesondere indem das Objekt mittels eines linken und eines rechten Teilstrahlengangs zeitlich alternierend auf den Bildsensor abgebildet wird, oder indem die beiden dem Rechts-Kanal und dem Links-Kanal entsprechenden Abbilder gleichzeitig nebeneinander auf dem Bildsensor erzeugt werden. Oder das Mikroskop umfasst zwei Bildsensoren, wobei mittels entsprechender Teilstrahlengänge das dem Rechts-Kanal entsprechende Abbild stets auf dem einen Bildsensor und das dem Links-Kanal entsprechende Abbild stets auf dem anderen Bildsensor erzeugt werden, und wobei die zwei Bildsensoren die stereoskopischen Bilddatensätze gleichzeitig erzeugen. Die Recheneinrichtung ist ferner dazu angepasst, aus den für die mehreren Fokuswerte erzeugten Primär-Bilddatensätzen einen jeweiligen Sekundär-Bilddatensatz des Rechts-Kanals und des Links-Kanals zu ermitteln, der relativ zu den jeweiligen Primär-Bilddatensätzen eine erweiterte Schärfentiefe aufweist. Der elektronische Sucher ist hierbei dazu angepasst, ein dem jeweiligen Sekundär-Bilddatensatz entsprechendes jeweiliges Bild des Rechts-Kanals und des Links-Kanals anzuzeigen. Somit wäre es prinzipiell denkbar, die vorstehend erläuterten Maßnahmen für zwei Stereo-Kanäle zumindest teilweise parallel durchzuführen, so dass am elektronischen Sucher stereoskopische (Sekundär-)Bilder angezeigt werden können.

Grundsätzlich können die Unterteilung in Bildbereiche und die Auswahl der jeweiligen Fokuslage bzw. des jeweiligen Primär-Bilddatensatzes für die beiden Stereo-Kanäle (Rechts und Links) identisch erfolgen. Dabei wäre es prinzipiell denkbar, die erläuterte Auswahl eines jeweiligen Primär-Bilddatensatzes für die beiden Stereo-Kanäle parallel und unabhängig voneinander durchzuführen.

Gemäß Anspruch 1 ist die Recheneinrichtung des Mikroskops jedoch dazu angepasst, den jeweiligen Sekundär-Bilddatensatz des Rechts-Kanals und des Links-Kanals dadurch zu ermitteln,
- dass die Primär-Bilddatensätze des Rechts-Kanals und die Primär-Bilddatensätze des Links-Kanals in mehrere, hinsichtlich der beiden Kanäle einander entsprechende (d.h. zumindest im Wesentlichen übereinstimmende) Bildbereiche unterteilt werden;
- dass die für die mehreren Fokuswerte erzeugten Primär-Bilddatensätze innerhalb der einzelnen Bildbereiche für lediglich einen der beiden Kanäle (d.h. lediglich für den Rechts-Kanal oder lediglich für den Links-Kanal) hinsichtlich des jeweiligen Schärfegrads ausgewertet werden, wobei zu jedem Bildbereich der Fokuswert mit dem höchsten Schärfegrad identifiziert wird (insbesondere in der bereits erläuterten Weise, z.B. durch Bilden und gegenseitiges Vergleichen von Differenzwerten); und
- dass für sowohl den Rechts-Kanal als auch den Links-Kanal der jeweilige Sekundär-Bilddatensatz dadurch ermittelt wird, dass für die mehreren Bildbereiche jeweils die Daten desjenigen Primär-Bilddatensatzes ausgewählt werden, der dem Fokuswert entspricht, welcher für den lediglich einen der beiden Kanäle identifiziert worden ist.

Somit wird die Ermittlung eines Schärfegrades und die entsprechende Auswahl des jeweiligen Primär-Bilddatensatzes lediglich für einen der beiden Stereo-Kanäle vorgenommen und das Auswahlergebnis automatisch auch für den anderen der beiden Stereo-Kanäle herangezogen. Mit anderen Worten wird ein wesentlicher Teil der Analyse (Ermittlung des Schärfegrads und entsprechende Auswahl des Fokuswerts bzw. der Fokuslage) lediglich für einen der beiden Stereo-Kanäle durchgeführt, wobei das Ergebnis auch für den anderen der beiden Stereo-Kanäle verwendet wird. Hierdurch kann der Rechenaufwand minimiert werden, und es lässt sich auch eine schnellere Verarbeitung der Bilddaten erreichen. Dies ist insbesondere auch für eine möglichst flüssige und verzögerungsfreie Wiedergabe der (Sekundär-)Bilder am elektronischen Sucher von Vorteil.

Die Erfindung beruht auf der Erkenntnis, dass bei einem Mikroskop zumindest bei manchen Anwendungen keine schnellen Bewegungsabläufe beobachtet werden müssen und auch keine schnellen Bewegungen der Kameraeinheit bzw. deren Abbildungsoptik (z.B. Schwenks) durchgeführt werden. Hierdurch besteht die Möglichkeit, in einem jeweiligen Sekundärbildermittlungszyklus in Abfolge und insbesondere in Echtzeit mehrere (Primär-)Bilder für unterschiedliche Fokuslagen (d.h. mit unterschiedlicher Lage der Fokusebene im Objektraum) zu erfassen und diese zu einem einzigen (Sekundär-)Bild mit erhöhter Schärfentiefe zu kombinieren und an dem elektronischen Sucher anzuzeigen. Diese Vorgehensweise ist insbesondere bei einem Einsatz des Mikroskops als Operationsmikroskop besonders vorteilhaft, da in diesem Anwendungsfall regelmäßig ein großer Schärfentiefebereich erwünscht ist, ohne dass hierfür eine Apertur der Abbildungsoptik unerwünscht stark verringert werden soll, was durch eine erhöhte Beleuchtungsintensität kompensiert werden müsste. Gleichzeitig stellt eine Operationsstelle oftmals eine vergleichsweise statische Szene dar, welche lediglich geringen zeitlichen Veränderungen unterliegt. Diese geringen zeitlichen Veränderungen werden zu einer bildverarbeitungstechnischen Generierung eines künstlichen Bildes mit erweiterter Schärfentiefe ausgenutzt, welches aus mehreren realen Bildern der Szene (mit jeweils unterschiedlicher Schärfentiefe bzw. Arbeitsabstand) zusammengesetzt wird.

Die im Zusammenhang mit der Erfindung begrifflich unterschiedenen Steuereinrichtung und die Recheneinrichtung können durch eine gemeinsame Einheit gebildet sein. Hierdurch ist die Anzahl der Einzelkomponenten des Mikroskops reduziert.

Ferner ist im Zusammenhang mit der Erfindung zu beachten, dass bei einer Ausbildung des elektronischen Mikroskops als Stereomikroskop das Erzeugen der Primär-Bilddatensätze und das Ermitteln eines jeweiligen Sekundär-Bilddatensatzes innerhalb eines Zyklus sowohl für einen Rechts-Kanal als auch für einen Links-Kanal durchgeführt werden können, wie nachstehend erläutert wird.

Gemäß einer Ausführungsform ist die Stelleinrichtung als eine elektrische, elektromagnetische, elektromotorische oder piezoelektrische Einrichtung ausgebildet. Hierdurch ist eine einfache und schnelle Ansteuerung möglich.

Um die Fokuslage der Kameraeinheit verändern zu können, kann die Abbildungsoptik beispielsweise wenigstens eine Linse aufweisen, die mittels der Stelleinrichtung entlang einer optischen Achse der Abbildungsoptik bewegbar ist.

Alternativ oder zusätzlich weist die Abbildungsoptik wenigstens eine Linse auf, deren Brennweite bzw. Brechkraft mittels der Stelleinrichtung veränderbar ist, um die Fokuslage der Kameraeinheit zu verändern.

Die Linse kann insbesondere als eine Flüssiglinse ausgebildet sein, um die Fokuslage besonders schnell verändern zu können. In diesem Fall ist die Stelleinrichtung z.B. als eine Elektrodenanordnung dergestalt ausgebildet, dass mittels einer, vorzugsweise zweier um die Linse angeordneter Ringelektroden ein elektrisches Feld an die Linse angelegt wird, wobei die Brennweite bzw. Brechkraft der Linse in Abhängigkeit von der Stärke des elektrischen Feldes veränderbar ist.

Gemäß einer weiteren Ausführungsform ist der Bildsensor mittels der Stelleinrichtung entlang einer optischen Achse der Abbildungsoptik bewegbar, um die Fokuslage der Kameraeinheit zu verändern. Diese Ausführungsform kann insbesondere alternativ zu dem vorherigen Fall einer mit der Abbildungsoptik zusammenwirkenden Stelleinrichtung angestrebt werden, z.B. wenn die Abbildungsoptik besonders kompakt oder gekapselt ausgebildet sein soll.

Die Stelleinrichtung kann einen oder mehrere Aktoren umfassen. Insbesondere kann die Stelleinrichtung dem Fachmann geläufige Aktoren von Kameraeinheiten z.B. zur Verstellung einer Blendenöffnung (Apertur) der Abbildungsoptik aufweisen.

Die Kameraeinheit und insbesondere deren Abbildungsoptik können für jeden der mehreren in einem Zyklus eingestellten Fokuswerte einen Schärfentiefebereich aufweisen, wobei die den mehreren Fokuswerten entsprechenden Fokuslagen der Kameraeinheit derart gewählt sind, dass die Schärfentiefebereiche der mehreren Fokuswerte paarweise überlappen. Hierdurch können die für die verschiedenen Fokuswerte erzeugten Primär-Bilddatensätze besonders gut zu einem Sekundär-Bilddatensatz mit einer durchgehend erweiterten Schärfentiefe kombiniert werden.

Insbesondere können sich die Fokuslagen der Kameraeinheit, die den eingestellten mehreren Fokuswerten entsprechen, jeweils um einen Abstand voneinander unterscheiden, der zumindest im Wesentlichen der Hälfte des jeweiligen Schärfentiefebereichs (d.h. des Schärfentiefebereichs eines jeweiligen Fokuswerts) entspricht. Ein entsprechendes gegenseitiges Überlappen der Schärfentiefebereiche der nacheinander eingestellten mehreren Fokuswerte kann z.B. dazu ausgenutzt werden, Variationen innerhalb eines jeweiligen Schärfentiefebereichs eines Primär-Bilddatensatzes oder zwischen den Primär-Bilddatensätzen zu kompensieren, sodass der effektive Schärfentiefebereich des ermittelten Sekundärbildes besonders homogen ist.

Die vorstehenden Angaben zu den Schärfentiefebereichen und den gegenseitigen Abständen beziehen sich generell auf eine Betrachtung entlang einer optischen Achse der Abbildungsoptik.

Nach einer bevorzugten Ausführungsform ist die Steuereinrichtung dazu angepasst, die Stelleinrichtung zu einem sich zyklisch wiederholenden Verändern der Fokuslage der Kameraeinheit zwischen wenigstens drei verschiedenen Fokuswerten anzusteuern und den Bildsensor zum Erzeugen eines jeweiligen Primär-Bilddatensatzes für jeden der wenigstens drei Fokuswerte anzusteuern. Die Recheneinrichtung ist hierbei dazu angepasst, aus den für die wenigstens drei Fokuswerte erzeugten Primär-Bilddatensätzen einen Sekundär-Bilddatensatz zu ermitteln, der relativ zu den jeweiligen Primär-Bilddatensätzen eine erweiterte Schärfentiefe aufweist. Durch die Erzeugung und Verwertung von drei verschiedenen Primär-Bilddatensätzen pro Sekundärbildermittlungszyklus kann für das jeweilige Sekundärbild eine ausreichend große und homogene Schärfentiefe erzielt werden, und gleichwohl sind ein Betrieb und insbesondere eine Wiedergabe der Sekundärbilder in dem elektronischen Sucher noch in Echtzeit oder nahezu in Echtzeit möglich.

Zusätzlich zu dem sich zyklisch wiederholenden Verändern der Fokuslage der Kameraeinheit kann die Fokuslage der Kameraeinheit für die mehreren Fokuswerte gemeinsam veränderbar sein. Hierdurch ist es möglich, den Arbeitsabstand des Mikroskops (Abstand zwischen der Abbildungsoptik und dem abzubildenden Objekt) zu variieren, wobei eine hiermit verbundene Anpassung der Fokuslage der Kameraeinheit mit dem zyklisch wiederholenden Verändern der Fokuslage überlagert wird. Gemäß einer besonders vorteilhaften Ausführungsform kann dies mittels derselben Stelleinrichtung geschehen. In diesem Fall können also mittels derselben Stelleinrichtung sowohl das zyklische Durchfahren der verschiedenen Fokuslagen zum Zwecke der Erzeugung der verschiedenen Primär-Bilddatensätze, als auch das (gegebenenfalls überlagerte) generelle Verstellen des Arbeitsabstands des Mikroskops erfolgen, ohne dass hierfür mehrere verschiedene Stelleinrichtungen erforderlich sind.

Nach einer weiteren Ausführungsform weist die Abbildungsoptik einen variablen Vergrößerungsfaktor (d.h. Abbildungsmaßstab, Zoom-Faktor) auf, wobei die Steuereinrichtung dazu angepasst ist, die Stelleinrichtung zu dem sich zyklisch wiederholenden Verändern der Fokuslage der Kameraeinheit für denselben Vergrößerungsfaktor anzusteuern. Mit anderen Worten kann auch bei einer Abbildungsoptik mit variablem Vergrößerungsfaktor das Erzeugen der Primär-Bilddatensätze für die verschiedenen Fokuslagen gleichwohl bei unverändertem Vergrößerungsfaktor erfolgen.

Zur Ermittlung des Sekundär-Bilddatensatzes ist die Recheneinrichtung vorzugsweise dazu angepasst, die für die mehreren Fokuswerte erzeugten Primär-Bilddatensätze in mehrere Bildbereiche zu unterteilen und innerhalb der Bildbereiche hinsichtlich eines jeweiligen Schärfegrads auszuwerten, sowie den Sekundär-Bilddatensatz dadurch zu ermitteln, dass für jeden der mehreren Bildbereiche die Daten des Primär-Bilddatensatzes mit dem höchsten Schärfegrad ausgewählt werden. Mit anderen Worten wird der Sekundär-Bilddatensatz aus den für die unterschiedlichen Bildbereiche ausgewählten Daten aufgrund des Kriteriums maximaler Schärfe zusammengesetzt.

Je nach Bildsensortyp kann ein jeweiliger Bildbereich eine Gruppe von mehreren räumlich benachbarten Bildelementen (Pixeln) derselben Farbe oder unterschiedlicher Farbe umfassen. Die Berücksichtigung von Bildelementen derselben Farbe ist insbesondere von Vorteil, wenn der Bildsensor Sensorelemente dreier verschiedener Farben aufweist, die nach dem Schema eines Farbmosaikfilters, vorzugsweise eines so genannten Bayer-Filters, angeordnet sind. In einem solchen Fall müssen nicht zwingend sämtliche Farben berücksichtigt werden, sondern es kann für die Ermittlung des jeweiligen Schärfegrads ausreichen, lediglich die Werte der Bildelemente einer einzigen Farbe (vorzugsweise Grün) miteinander zu vergleichen und/oder zu verrechnen, wobei die Bildelemente der weiteren Farben ohne weitere Prüfung genauso behandelt werden wie die benachbarten Bildelemente der tatsächlich berücksichtigten Farbe. Der genannte jeweilige Bildbereich kann im Extremfall auch durch ein einziges Bildelement oder lediglich zwei Bildelemente gebildet sein. Es sind jedoch stets mehrere Bildbereiche vorgesehen, d.h. das (Sekundär-) Bild setzt sich aus mehreren Bildbereichen bzw. den für mehrere Bildbereiche ermittelten Sekundär-Bilddaten zusammen.

Die betrachteten Bildelemente können direkt oder indirekt benachbart sein. Letzteres ist insbesondere der Fall, wenn bei einem Bildsensor mit Farbmosaikfilter - wie erläutert - lediglich die Bilddaten von Bildelementen jeweils derselben Farbe herangezogen werden. Der jeweilige Bildbereich umfasst jedoch eine räumliche Umgebung von Bildelementen, die eine rechnerische Ermittlung und auch eine visuelle Wahrnehmung eines Schärfegrads ermöglicht.

Die Recheneinrichtung kann dazu angepasst sein, die Primär-Bilddatensätze hinsichtlich des Schärfegrads dadurch auszuwerten, dass für den jeweiligen Bildbereich basierend auf dem jeweiligen Primär-Bilddatensatz (und insbesondere für die Bildelemente derselben Farbe) wenigstens eine Differenz oder wenigstens ein Quotient zwischen den Bilddaten wenigstens zweier benachbarter Bildelemente (d.h. zwischen den betreffenden Bildelement-Werten) gebildet wird. Je größer die Differenz oder der Quotient, umso größer ist generell der Schärfegrad. Somit ist es für das Ermitteln des Sekundär-Bilddatensatzes insbesondere möglich, dass für jeden der unterschiedlichen Bildbereiche die Daten des Primär-Bilddatensatzes mit der größten Differenz bzw. mit dem größten Quotienten zwischen den Bildelement-Werten ausgewählt werden. Hierbei können für einen jeweiligen Bildbereich auch mehrere Differenzen oder Quotienten gebildet werden, wobei die hierdurch erzeugten Werte beispielsweise gemittelt werden können, um einen Schärfegrad zu ermitteln.

Es kann eine absolute Differenz oder eine normierte Differenz gebildet werden, d.h. die Differenz wird durch einen Absolutwert dividiert.

Das gegenseitige Verrechnen der betrachteten Bildelemente bzw. Bildbereiche muss nicht linear, sondern kann auch in höherer Ordnung erfolgen. Ferner können die betrachteten Bildelemente in einer einzigen Richtung oder in mehreren Richtungen zueinander benachbart sein (z.B. horizontal und/oder vertikal).

Gemäß einer weiteren Ausführungsform sind die Steuereinrichtung, die Recheneinrichtung und der elektronische Sucher zueinander synchronisiert und dazu angepasst,
- das Erzeugen der Primär-Bilddatensätze für die mehreren Fokuswerte eines Zyklus,
- das Ermitteln des jeweiligen Sekundär-Bilddatensatzes und
- das Anzeigen des jeweiligen (Sekundär-)Bildes
mit einer Frequenz durchzuführen, die wenigstens 25 Hz beträgt. Hierdurch wird gewährleistet, dass das dem Sekundär-Bilddatensatz entsprechende Bild zumindest subjektiv im Wesentlichen kontinuierlich ("ruckelfrei") und mit möglichst geringer Verzögerung dem Benutzer des Mikroskops über den elektronischen Sucher angezeigt werden kann.

Der elektronische Sucher kann insbesondere zwei elektronische Anzeigeeinrichtungen oder eine in zwei Anzeigebereiche unterteilte elektronische Anzeigeeinrichtung aufweisen, wobei der elektronische Sucher dazu angepasst ist, das jeweilige (Sekundär-)Bild des Rechts-Kanals und des Links-Kanals an den zwei Anzeigeeinrichtungen oder zwei Anzeigebereichen anzuzeigen. Die Bilder des Rechts-Kanals und des Links-Kanals können von dem Benutzer des Mikroskops über ein jeweils zugeordnetes Okular betrachtet werden, wobei das Bild des Rechts-Kanals ausschließlich dem rechten und das Bild des Links-Kanals ausschließlich dem linken Auge zugeführt wird.

Nach einer besonders vorteilhaften Ausführungsform ist das elektronische Mikroskop wahlweise in einer ersten Betriebsart erweiterter Schärfentiefe oder in einer zweiten Betriebsart erhöhter zeitlicher Auflösung betreibbar. In der ersten Betriebsart werden - wie vorstehend im Einzelnen erläutert - die Sekundär-Bilddatensätze aus den für die mehreren Fokuswerte erzeugten Primär-Bilddatensätzen ermittelt und an dem elektronischen Sucher als (Sekundär-)Bild angezeigt (ggf. stereoskopisch). In der zweiten Betriebsart ist die Steuereinrichtung dazu angepasst, den oder die Bildsensor(en) zum Erzeugen von Primär-Bilddatensätzen anzusteuern, ohne die Stelleinrichtung zu einem sich zyklisch wiederholenden Verändern der Fokuslage der Kameraeinheit zwischen den mehreren Fokuswerten anzusteuern. Mit anderen Worten wird in der zweiten Betriebsart zugunsten einer schnelleren Bilderzeugung darauf verzichtet, mehrere Fokuslagen zu durchfahren, um die Schärfentiefe zu vergrößern. Hierbei ist der elektronische Sucher dazu angepasst, ein dem jeweiligen Primär-Bilddatensatz entsprechendes (Primär-)Bild anzuzeigen. In dieser Ausführungsform kann der Benutzer dem Betrieb des Mikroskops also wahlweise entweder eine erweiterte Schärfentiefe oder eine erhöhte zeitliche Auflösung zugrunde legen, wobei ein schnelles Umschalten zwischen den beiden Betriebsarten vorgesehen sein kann. Ferner ist es auch denkbar, eine graduelle Einstellung dieser miteinander konkurrierenden Betriebskriterien vorzusehen (z.B. mit einer variablen Anzahl von Fokuswerten und entsprechenden Primär-Bilddatensätzen, die pro Zyklus eingestellt bzw. ausgewertet werden).

Die Erfindung wird im Folgenden lediglich beispielhaft anhand einer Ausführungsform beschrieben. Es zeigt:
- Fig. 1: eine Perspektivansicht eines Mikroskops mit Stativ; und
- Fig. 2: eine schematische Längsschnittansicht eines Mikroskops.

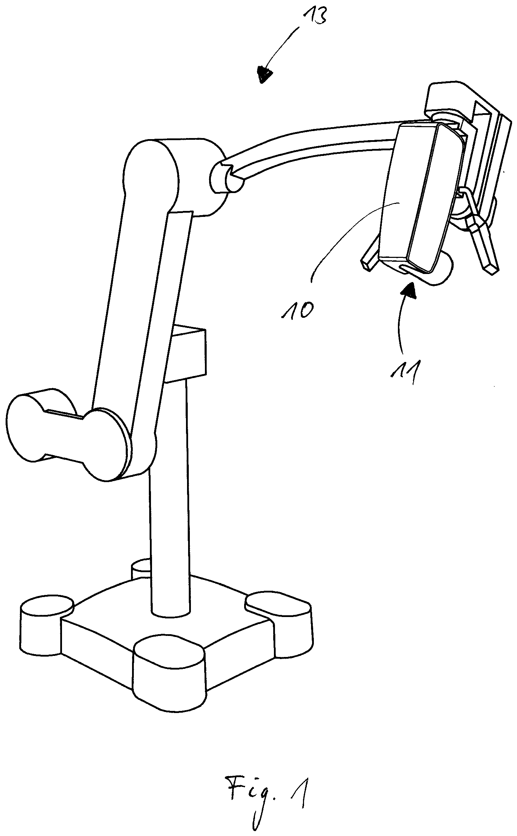

Fig. 1 zeigt ein elektronisches stereoskopisches Mikroskop 10 in einer Anwendung als Operationsmikroskop. Das Mikroskop 10 umfasst eine Kameraeinheit 11, die an einem mehrachsig schwenkbaren Stativ 13 befestigt ist und bei dem gezeigten Ausführungsbeispiel abgewinkelt ausgeführt ist, wie dies aus DE 10 2012 218 863 A1 und DE 10 2013 208 306 A1 bekannt ist. Das Mikroskop 10 umfasst ferner einen der Kameraeinheit 11 zugeordneten elektronischen Sucher (in Fig. 1 nicht dargestellt). Ein derartiges Mikroskop 10 kommt beispielsweise bei chirurgischen Eingriffen im Bereich der klinischen Medizin zum Einsatz.

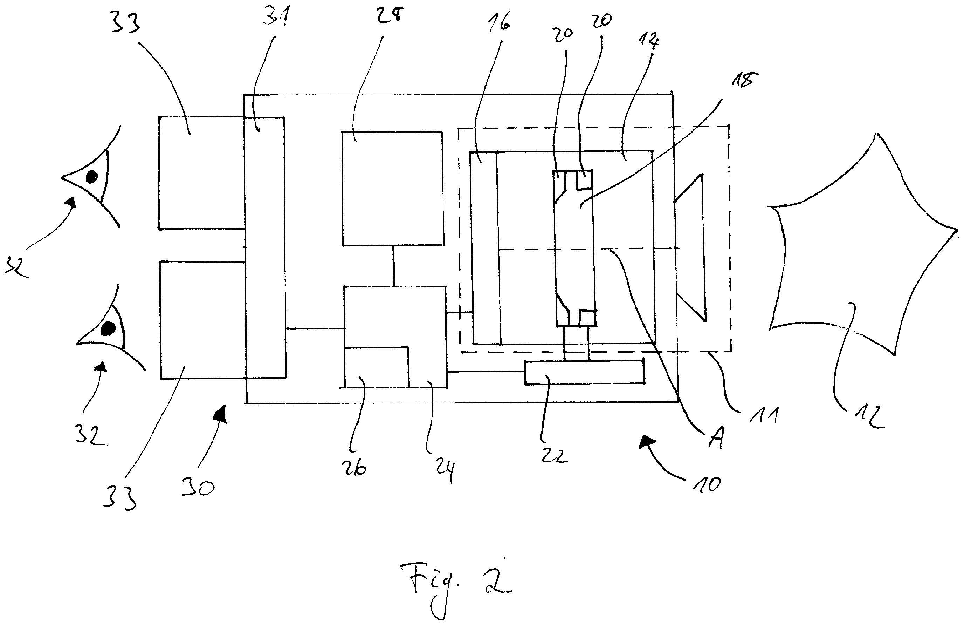

Fig. 2 zeigt Einzelheiten eines derartigen elektronischen Mikroskops 10 in einer geradlinigen Ausführung, wobei zum Zwecke einer einfacheren Darstellung ein einziger (d.h. monoskopischer) Strahlengang gezeigt ist. Das Mikroskop 10 weist zur optischen Erfassung einer Operationsstelle 12 an der Kameraeinheit 11 eine Abbildungsoptik 14 mit einer optischen Achse A, sowie zumindest einen elektronischen Bildsensor 16 auf, der in einer zweidimensionalen Anordnung eine Vielzahl von lichtempfindlichen Sensorelementen umfasst. Die Abbildungsoptik 14 umfasst eine Flüssiglinse 18, deren Brennweite variabel einstellbar ist. Hierzu ist eine Stelleinrichtung 22 vorgesehen, welche zwei um die Flüssiglinse 18 umlaufende Elektroden 20 aufweist. Über die Elektroden 20 ist ein elektrisches Feld erzeugbar, das eine erwünschte Einstellung der Brennweite der Flüssiglinse 18 bewirkt.

In einer hier nicht gezeigten stereoskopischen Ausführungsform umfasst das Mikroskop 10 einen gleichartigen zweiten Strahlengang, um einen zweiten Kanal bereitzustellen und somit jeweilige Primär-Bilddatensätze eines Rechts-Kanals und eines Links-Kanals erzeugen zu können. Die Strahlengänge des Rechts-Kanals und des Links-Kanals können getrennte oder zumindest teilweise identische Komponenten aufweisen (z.B. gemeinsame optische Elemente, eine gemeinsame Stelleinrichtung 22 und/oder einen gemeinsamen Bildsensor 16), wobei vorzugsweise eine gemeinsame Stelleinrichtung 22 zum Verändern der Fokuslage der beiden Kanäle der Kameraeinheit 11 vorgesehen ist.

Die Stelleinrichtung 22 wird von einer mit der Stelleinrichtung 22 verbundenen Steuereinrichtung 24 gesteuert. Die Steuereinrichtung 24 dient darüber hinaus zur Steuerung des Bildsensors 16, einer Recheneinrichtung 28 sowie eines elektronischen Suchers 30 des Mikroskops 10. Der elektronische Sucher 30 umfasst eine zweigeteilte elektronische Anzeigeeinrichtung 31 sowie zwei auf die Anzeigeeinrichtung 31 aufgesetzte Okulare 33. Konfigurationsdaten des Mikroskops 10 sind in einem Speicher 26 der Steuereinrichtung 24 abgelegt, der zugleich als Bilddatenspeicher dient.

In einer Betriebsart erweiterter Schärfentiefe des Mikroskops 10 steuert die Steuereinrichtung 24 die Stelleinrichtung 22 in Abhängigkeit der in dem Speicher 26 abgelegten Konfigurationsdaten in schneller Abfolge zu einem sich zyklisch wiederholenden Verändern der Fokuslage der Abbildungsoptik 14 gemäß mehreren verschiedenen Brennweitenwerten der Flüssiglinse 18 an. Für jede entsprechend den mehreren Brennweitenwerten eingestellte Fokuslage steuert die Steuereinrichtung 24 den Bildsensor 16 zum Erzeugen eines jeweils zugeordneten Primär-Bilddatensatzes an, welcher in dem Speicher 26 der Steuereinrichtung 24 gespeichert wird.

Bei Vollendung eines jeweiligen Zyklus ermittelt die Recheneinrichtung 28 aus den im Zuge eines Zyklus erzeugten Primär-Bilddatensätzen - wie vorstehend bereits erläutert - einen Sekundär-Bilddatensatz, der relativ zu den jeweiligen Primär-Bilddatensätzen eine erweiterte Tiefenschärfe aufweist. Das dem ermittelten Sekundär-Bilddatensatz entsprechende Bild wird an dem elektronischen Sucher 30 angezeigt und ist durch die Okulare 33 von einem Paar menschlicher Augen 32 eines nicht näher gezeigten Benutzers des Mikroskops 10 zu betrachten. Das angezeigte Bild erlaubt es dem Benutzer, die Operationsstelle 12 "live" ohne signifikante Zeitverzögerung mit einer großen Schärfentiefe zu verfolgen. Durch Verwendung des Mikroskops 10 kann die Operationsstelle 12 mit einer gewünschten Vergrößerung dargestellt werden. Ein Chirurg kann die Operation also mit hoher Präzision und geringer Ermüdung durchführen.

### Bezugszeichenliste

- 10: Mikroskop
- 11: Kameraeinheit
- 13: Stativ
- 12: Operationsstelle
- 14: Abbildungsoptik
- 16: Bildsensor
- 18: Flüssiglinse
- 20: Elektrode
- 22: Stelleinrichtung
- 24: Steuereinrichtung
- 26: Speicher
- 28: Recheneinrichtung
- 30: Sucher
- 31: Anzeigeeinrichtung
- 32: Auge
- 33: Okular
- A: optische Achse

## Patentansprüche

1. Elektronisches Mikroskop (10) mit einer Kameraeinheit (11), die zumindest einen elektronischen Bildsensor (16) zum Erzeugen von Primär-Bilddatensätzen und eine Abbildungsoptik (14) zum Erzeugen eines Abbildes eines Objekts (12) auf dem Bildsensor (16) umfasst;
einem elektronischen Sucher (30);
einer Stelleinrichtung (22) zum Verändern einer Fokuslage der Kameraeinheit (11);
einer Steuereinrichtung (24); und
einer Recheneinrichtung (28);
wobei die Steuereinrichtung (24) dazu angepasst ist, die Stelleinrichtung (22) zu einem sich zyklisch wiederholenden Verändern der Fokuslage der Kameraeinheit (11) zwischen mehreren Fokuswerten anzusteuern und den Bildsensor (16) zum Erzeugen eines jeweiligen Primär-Bilddatensatzes für jeden der mehreren Fokuswerte anzusteuern;
wobei die Recheneinrichtung (28) dazu angepasst ist, aus den für die mehreren Fokuswerte erzeugten Primär-Bilddatensätzen einen Sekundär-Bilddatensatz zu ermitteln, der relativ zu den jeweiligen Primär-Bilddatensätzen eine erweiterte Schärfentiefe aufweist; und
wobei der elektronische Sucher (30) dazu angepasst ist, ein dem Sekundär-Bilddatensatz entsprechendes Bild anzuzeigen;
wobei das elektronische Mikroskop (10) als ein Stereomikroskop ausgebildet ist, wobei die Steuereinrichtung (24) dazu angepasst ist, für jeden der mehreren Fokuswerte den zumindest einen Bildsensor (16) zum Erzeugen eines jeweiligen Primär-Bilddatensatzes eines Rechts-Kanals und eines Links-Kanals anzusteuern;
wobei die Recheneinrichtung (28) dazu angepasst ist, aus den für die mehreren Fokuswerte erzeugten Primär-Bilddatensätzen einen jeweiligen Sekundär-Bilddatensatz des Rechts-Kanals und des Links-Kanals zu ermitteln, der relativ zu den jeweiligen Primär-Bilddatensätzen eine erweiterte Schärfentiefe aufweist;
wobei der elektronische Sucher (30) dazu angepasst ist, ein dem jeweiligen Sekundär-Bilddatensatz entsprechendes jeweiliges Bild des Rechts-Kanals und des Links-Kanals anzuzeigen,
**dadurch gekennzeichnet, dass**
das elektronische Mikroskop als ein Operationsmikroskop ausgebildet ist, wobei die Recheneinrichtung (28) ferner dazu angepasst ist, den jeweiligen Sekundär-Bilddatensatz des Rechts-Kanals und des Links-Kanals dadurch zu ermitteln, dass
- die Primär-Bilddatensätze des Rechts-Kanals und die Primär-Bilddatensätze des Links-Kanals in mehrere, hinsichtlich der beiden Kanäle einander entsprechende Bildbereiche unterteilt werden;
- die für die mehreren Fokuswerte erzeugten Primär-Bilddatensätze innerhalb der Bildbereiche für lediglich einen der beiden Kanäle hinsichtlich eines jeweiligen Schärfegrads ausgewertet werden, wobei der Fokuswert mit dem höchsten Schärfegrad identifiziert wird; und
- für sowohl den Rechts-Kanal als auch den Links-Kanal der jeweilige Sekundär-Bilddatensatz dadurch ermittelt wird, dass für die mehreren Bildbereiche die Daten desjenigen Primär-Bilddatensatzes ausgewählt werden, der dem für den einen der beiden Kanäle identifizierten Fokuswert entspricht.

2. Elektronisches Mikroskop (10) nach Anspruch 1,
wobei die Stelleinrichtung (22) als eine elektrische, elektromagnetische, elektromotorische oder piezoelektrische Einrichtung ausgebildet ist.

3. Elektronisches Mikroskop (10) nach Anspruch 1 oder 2,
wobei die Abbildungsoptik (14) wenigstens eine Linse (18) aufweist, die mittels der Stelleinrichtung (22) entlang einer optischen Achse (A) der Abbildungsoptik (14) bewegbar ist, um die Fokuslage der Kameraeinheit (11) zu verändern.

4. Elektronisches Mikroskop (10) nach zumindest einem der vorhergehenden Ansprüche,
wobei die Abbildungsoptik (14) wenigstens eine Linse (18) aufweist, deren Brennweite mittels der Stelleinrichtung (22) veränderbar ist, um die Fokuslage der Kameraeinheit (11) zu verändern.

5. Elektronisches Mikroskop (10) nach zumindest einem der vorhergehenden Ansprüche,
wobei der Bildsensor (16) mittels der Stelleinrichtung (22) entlang einer optischen Achse (A) der Abbildungsoptik (14) bewegbar ist, um die Fokuslage der Kameraeinheit (11) zu verändern.

6. Elektronisches Mikroskop (10) nach zumindest einem der vorhergehenden Ansprüche,
wobei die Kameraeinheit (11) für jeden der mehreren Fokuswerte einen Schärfentiefebereich aufweist, wobei die den mehreren Fokuswerten entsprechenden Fokuslagen derart gewählt sind, dass die Schärfentiefebereiche der mehreren Fokuswerte paarweise überlappen;
wobei die den mehreren Fokuswerten entsprechenden Fokuslagen sich um einen Abstand unterscheiden, der zumindest im Wesentlichen der Hälfte des jeweiligen Schärfentiefebereichs entspricht.

7. Elektronisches Mikroskop (10) nach zumindest einem der vorhergehenden Ansprüche,
wobei die Steuereinrichtung (24) dazu angepasst ist, die Stelleinrichtung (22) zu einem sich zyklisch wiederholenden Verändern der Fokuslage der Kameraeinheit (11) zwischen wenigstens drei verschiedenen Fokuswerten anzusteuern und den Bildsensor zum Erzeugen eines jeweiligen Primär-Bilddatensatzes für jeden der wenigstens drei Fokuswerte anzusteuern; wobei die Recheneinrichtung (28) dazu angepasst ist, aus den für die wenigstens drei Fokuswerte erzeugten Primär-Bilddatensätzen einen Sekundär-Bilddatensatz zu ermitteln, der relativ zu den jeweiligen Primär-Bilddatensätzen eine erweiterte Schärfentiefe aufweist.

8. Elektronisches Mikroskop (10) nach zumindest einem der vorhergehenden Ansprüche,
wobei zusätzlich zu dem sich zyklisch wiederholenden Verändern der Fokuslage der Kameraeinheit (11) die Fokuslage der Kameraeinheit (11) für die mehreren Fokuswerte gemeinsam veränderbar ist.

9. Elektronisches Mikroskop (10) nach zumindest einem der vorhergehenden Ansprüche,
wobei die Abbildungsoptik (14) einen variablen Vergrößerungsfaktor aufweist, wobei die Steuereinrichtung (24) dazu angepasst ist, die Stelleinrichtung (22) zu dem sich zyklisch wiederholenden Verändern der Fokuslage der Kameraeinheit (11) für denselben Vergrößerungsfaktor anzusteuern.

10. Elektronisches Mikroskop (10) nach zumindest einem der vorhergehenden Ansprüche,
wobei die Recheneinrichtung (28) dazu angepasst ist, die für die mehreren Fokuswerte erzeugten Primär-Bilddatensätze in mehrere Bildbereiche zu unterteilen und innerhalb der Bildbereiche hinsichtlich eines jeweiligen Schärfegrads auszuwerten, sowie den Sekundär-Bilddatensatz dadurch zu ermitteln, dass für jeden der mehreren Bildbereiche die Daten des Primär-Bilddatensatzes mit dem höchsten Schärfegrad ausgewählt werden.

11. Elektronisches Mikroskop (10) nach Anspruch 10,
wobei die Recheneinrichtung (28) dazu angepasst ist, die Primär-Bilddatensätze dadurch hinsichtlich des Schärfegrads auszuwerten, dass für den jeweiligen Bildbereich basierend auf dem jeweiligen Primär-Bilddatensatz wenigstens eine Differenz oder wenigstens ein Quotient zwischen den Bilddaten wenigstens zweier benachbarter Bildelemente gebildet wird.

12. Elektronisches Mikroskop (10) nach zumindest einem der vorhergehenden Ansprüche,
wobei die Steuereinrichtung (24), die Recheneinrichtung (28) und der elektronische Sucher (30) zueinander synchronisiert und dazu angepasst sind,
- das Erzeugen der Primär-Bilddatensätze für die mehreren Fokuswerte eines Zyklus,
- das Ermitteln des jeweiligen Sekundär-Bilddatensatzes und
- das Anzeigen des jeweiligen Bildes
mit einer Frequenz durchzuführen, die wenigstens 25 Hz beträgt.

13. Elektronisches Mikroskop (10) nach zumindest einem der vorhergehenden Ansprüche,
wobei der elektronische Sucher (30) zwei elektronische Anzeigeeinrichtungen (31) oder eine in zwei Anzeigebereiche unterteilte elektronische Anzeigeeinrichtung (31) aufweist, wobei der elektronische Sucher (30) dazu angepasst ist, das jeweilige Bild des Rechts-Kanals und des Links-Kanals an den zwei Anzeigeeinrichtungen (31) oder zwei Anzeigebereichen anzuzeigen.

14. Elektronisches Mikroskop (10) nach zumindest einem der vorhergehenden Ansprüche,
wobei das elektronische Mikroskop (10) wahlweise in einer ersten Betriebsart erweiterter Schärfentiefe oder in einer zweiten Betriebsart erhöhter zeitlicher Auflösung betreibbar ist;
wobei in der ersten Betriebsart die Sekundär-Bilddatensätze aus den für die mehreren Fokuswerte erzeugten Primär-Bilddatensätzen ermittelt und an dem elektronischen Sucher (30) angezeigt werden; und
wobei in der zweiten Betriebsart die Steuereinrichtung (24) dazu angepasst ist, den Bildsensor (16) zum Erzeugen von Primär-Bilddatensätzen anzusteuern, ohne die Stelleinrichtung (22) zu einem sich zyklisch wiederholenden Verändern der Fokuslage der Kameraeinheit zwischen den mehreren Fokuswerten anzusteuern, und der elektronische Sucher (30) dazu angepasst ist, ein dem jeweiligen Primär-Bilddatensatz entsprechendes Bild anzuzeigen.

## Claims

1. An electronic microscope (10) having a camera unit (11) which comprises at least one electronic image sensor (16) for generating primary image data sets and which comprises an imaging optics (14) for generating an image of an object (12) on the image sensor (16);
an electronic viewfinder (30);
an adjustment device (22) for varying a focal position of the camera unit (11);
a control device (24); and
a computing device (28),
wherein the control device (24) is adapted to control the adjustment device (22) to make a cyclically repeating variation of the focal position of the camera unit (11) between a plurality of focal values and to control the image sensor (16) to generate a respective primary image data set for each of the plurality of focal values;
wherein the computing device (28) is adapted to determine a secondary image data set from the primary image data sets generated for the plurality of focal values, said secondary image data set having an extended depth of field relative to the respective primary image data sets;
wherein the electronic viewfinder (30) is adapted to display an image corresponding to the secondary image data set;
wherein the electronic microscope (10) is configured as a stereo microscope, with the control device (24) being adapted to control the at least one image sensor (16) to generate a respective primary image data set of a right channel and of a left channel for each of the plurality of focal values;
wherein the computing device (28) is adapted to determine a respective secondary image data set of the right channel and of the left channel from the primary image data sets generated for the plurality of focal values, said secondary image data set having an extended depth of field relative to the respective primary image data sets; and
wherein the electronic viewfinder (30) is adapted to display a respective image of the right channel and of the left channel corresponding to the respective secondary image data set,
**characterized in that**
the electronic microscope is configured as a surgical microscope, with the computing device (28) further being adapted to determine the respective secondary image data set of the right channel and of the left channel **in that**
- the primary image data sets of the right channel and the primary image data sets of the left channel are divided into a plurality of image zones corresponding to one another with respect to the two channels;
- the primary image data sets generated for the plurality of focal values are evaluated within the image zones for only one of the two channels with respect to a respective degree of sharpness, with the focal value having the highest degree of sharpness being identified; and
- the respective secondary image data set for both the right channel and the left channel is determined by selecting for the plurality of image zones the data of that primary image data set that corresponds to the focal value identified for the one of the two channels.

2. An electronic microscope (10) in accordance with claim 1,
wherein the adjustment device (22) is configured as an electrical, electromagnetic, electromotoric or piezoelectric device.

3. An electronic microscope (10) in accordance with claim 1 or claim 2, wherein the imaging optics (14) has at least one lens (18) which is movable along an optical axis (A) of the imaging optics (14) by means of the adjustment device (22) to vary the focal position of the camera unit (11).

4. An electronic microscope (10) in accordance with at least one of the preceding claims,
wherein the imaging optics (14) has at least one lens (18) whose focal length is variable by means of the adjustment device (22) to vary the focal position of the camera unit (11).

5. An electronic microscope (10) in accordance with at least one of the preceding claims,
wherein the image sensor (16) is movable along an optical axis (A) of the imaging optics (14) by means of the adjustment device (22) to vary the focal position of the camera unit (11).

6. An electronic microscope (10) in accordance with at least one of the preceding claims,
wherein the camera unit (11) has a depth of field zone for each of the plurality of focal values, with the focal positions corresponding to the plurality of focal values being selected such that the depth of field zones of the plurality of focal values overlap pair-wise; and
wherein the focal positions corresponding to the plurality of focal values differ by a distance which at least substantially corresponds to half the respective depth of field zone.

7. An electronic microscope (10) in accordance with at least one of the preceding claims,
wherein the control device (24) is adapted to control the adjustment device (22) to make a cyclically repeating variation of the focal position of the camera unit (11) between at least three different focal values and to control the image sensor to generate a respective primary image data set for each of the at least three focal values;
wherein the computing device (28) is adapted to determine a secondary image data set from the primary image data sets generated for the at least three focal values, said secondary image data set having an extended depth of field relative to the respective primary image data sets.

8. An electronic microscope (10) in accordance with at least one of the preceding claims,
wherein, in addition to the cyclically repeating variation of the focal position of the camera unit (11), the focal position of the camera unit (11) for the plurality of focal values can also be varied together.

9. An electronic microscope (10) in accordance with at least one of the preceding claims,
wherein the imaging optics (14) has a variable magnification factor, with the control device (24) being adapted to control the adjustment device (22) to make the cyclically repeating variation of the focal position of the camera unit (11) for the same magnification factor.

10. An electronic microscope (10) in accordance with at least one of the preceding claims,
wherein the computing device (28) is adapted to divide the primary image data sets generated for the plurality of focal values into a plurality of image zones and to evaluate them within the image zones with respect to a respective degree of sharpness and to determine the secondary image data set in that the data of the primary image data set having the highest degree of sharpness for each of the plurality of image zones are selected.

11. An electronic microscope (10) in accordance with claim 10,
wherein the computing device (28) is adapted to evaluate the primary image data sets with respect to the degree of sharpness such that at least one difference or at least one quotient is formed between the image data of at least two adjacent picture elements for the respective image zone on the basis of the respective primary image data set.

12. An electronic microscope (10) in accordance with at least one of the preceding claims,
wherein the control device (24), the computing device (28) and the electronic viewfinder (30) are synchronized with one another and are adapted
- to generate the primary image data sets for the plurality of focal values of a cycle;
- to determine the respective secondary image data set; and
- to display the respective image
at a frequency which amounts to at least 25 Hz.

13. An electronic microscope (10) in accordance with at least one of the preceding claims,
wherein the electronic viewfinder (30) has two electronic display devices (31) or one electronic display device (31) divided into two display zones, with the electronic viewfinder (30) being adapted to display the respective image of the right channel and of the left channel at the two display devices (31) or at the two display zones.

14. An electronic microscope (10) in accordance with at least one of the preceding claims,
wherein the electronic microscope (10) is selectively operable in a first operating mode of extended depth of field or in a second operating mode of increased temporal resolution;
wherein, in the first operating mode, the secondary image data sets are determined from the primary image data sets generated for the plurality of focal values and are displayed at the electronic viewfinder (30); and wherein, in the second operating mode, the control device (24) is adapted to control the image sensor (16) to generate primary image data sets without controlling the adjustment device (22) to make a cyclically repeating variation of the focal position of the camera unit between the plurality of focal values, and the electronic viewfinder (30) is adapted to display an image corresponding to the respective primary image data set.

## Revendications

1. Microscope électronique (10) comportant une unité de caméra (11) qui comprend au moins un capteur d'image électronique (16) pour générer des ensembles de données d'image primaire et une optique d'imagerie (14) pour générer une image d'un objet (12) sur le capteur d'image (16) ;
un viseur électronique (30);
un dispositif de réglage (22) pour modifier une position focale de l'unité de caméra (11) ;
un dispositif de commande (24) ; et
un dispositif de calcul (28) ;
dans lequel
le dispositif de commande (24) est adapté à piloter le dispositif de réglage (22) en vue d'une modification répétée cycliquement de la position focale de l'unité de caméra (11) entre plusieurs valeurs focales, et à piloter le capteur d'image (16) en vue de générer un ensemble respectif de données d'image primaire pour chacune desdites plusieurs valeurs focales ;
le dispositif de calcul (28) est adapté à déterminer, à partir des ensembles de données d'image primaire générés pour lesdites plusieurs valeurs focales, un ensemble de données d'image secondaire qui présente une profondeur de champ étendue par rapport aux ensembles respectifs de données d'image primaire ; et
le viseur électronique (30) est adapté à afficher une image correspondante à l'ensemble de données d'image secondaire ;
le microscope électronique (10) est réalisé sous forme de stéréomicroscope, le dispositif de commande (24) étant adapté à piloter, pour chacune desdites plusieurs valeurs focales, ledit au moins un capteur d'image (16) en vue de générer un ensemble respectif de données d'image primaire d'un canal de droite et d'un canal de gauche ;
le dispositif de calcul (28) est adapté à déterminer, à partir des ensembles de données d'image primaire générés pour lesdites plusieurs valeurs focales, un ensemble respectif de données d'image secondaire du canal de droite et du canal de gauche, qui présente une profondeur de champ étendue par rapport aux ensembles respectifs de données d'image primaire ; le viseur électronique (30) est adapté à afficher une image respective du canal de droite et du canal de gauche, correspondante à l'ensemble respectif de données d'image secondaire,
**caractérisé en ce que**
le microscope électronique est réalisé sous forme de microscope chirurgical, le dispositif de calcul (28) étant en outre adapté à déterminer l'ensemble respectif de données d'image secondaire du canal de droite et du canal de gauche par le fait que
- les ensembles de données d'image primaires du canal de droite et les ensembles de données d'image primaire du canal de gauche sont subdivisés en plusieurs zones d'image qui se correspondent à l'égard des deux canaux ;
- les ensembles de données d'image primaire générés pour lesdites plusieurs valeurs focales à l'intérieur des zones d'image sont évalués, quant à un niveau de netteté respectif, uniquement pour l'un des deux canaux, la valeur focale ayant le plus haut niveau de netteté étant identifiée ; et
- aussi bien pour le canal de droite que pour le canal de gauche, l'ensemble respectif de données d'image secondaire est déterminé du fait que pour lesdites plusieurs zones d'image, les données de cet ensemble de données d'image primaire sont sélectionnées qui correspondent à la valeur focale identifiée pour l'un des deux canaux.

2. Microscope électronique (10) selon la revendication 1,
dans lequel
le dispositif de réglage (22) est réalisé sous forme de dispositif électrique, électromagnétique, électromoteur ou piézoélectrique.

3. Microscope électronique (10) selon la revendication 1 ou 2,
dans lequel
l'optique d'imagerie (14) comprend au moins une lentille (18) qui est mobile le long d'un axe optique (A) de l'optique d'imagerie (14) au moyen du dispositif de réglage (22), afin de modifier la position focale de l'unité de caméra (11).

4. Microscope électronique (10) selon l'une au moins des revendications précédentes,
dans lequel
l'optique d'imagerie (14) comprend au moins une lentille (18) dont la focale est variable au moyen du dispositif de réglage (22), afin de modifier la position focale de l'unité de caméra (11).

5. Microscope électronique (10) selon l'une au moins des revendications précédentes,
dans lequel
le capteur d'image (16) est mobile le long d'un axe optique (A) de l'optique d'imagerie (14) au moyen du dispositif de réglage (22), afin de modifier la position focale de l'unité de caméra (11).

6. Microscope électronique (10) selon l'une au moins des revendications précédentes,
dans lequel
l'unité de caméra (11) présente une plage de profondeur de champ pour chacune desdites plusieurs valeurs focales, les positions focales correspondant auxdites plusieurs valeurs focales étant choisies de telle sorte que les plages de profondeur de champ desdites plusieurs valeurs focales se chevauchent par paire ;
les positions focales qui correspondent auxdites plusieurs valeurs focales diffèrent d'une distance qui correspond au moins sensiblement à la moitié de la plage de profondeur de champ respective.

7. Microscope électronique (10) selon l'une au moins des revendications précédentes,
dans lequel
le dispositif de commande (24) est adapté à piloter le dispositif de réglage (22) en vue d'une modification répétée cycliquement de la position focale de l'unité de caméra (11) entre au moins trois valeurs focales différentes, et à piloter le capteur d'image en vue de générer un ensemble respectif de données d'image primaire pour chacune desdites au moins trois valeurs focales ;
le dispositif de calcul (28) est adapté à déterminer, à partir des ensembles de données d'image primaire générés pour lesdites au moins trois valeurs focales, un ensemble de données d'image secondaire qui présente une profondeur de champ étendue par rapport aux ensembles respectifs de données d'image primaire.

8. Microscope électronique (10) selon l'une au moins des revendications précédentes,
dans lequel
en supplément à la modification répétée cycliquement de la position focale de l'unité de caméra (11), la position focale de l'unité de caméra (11) est variable conjointement pour lesdites plusieurs valeurs focales.

9. Microscope électronique (10) selon l'une au moins des revendications précédentes,
dans lequel
l'optique d'imagerie (14) présente un facteur de grossissement variable, le dispositif de commande (24) étant adapté à piloter le dispositif de réglage (22) en vue de la modification répétée cycliquement de la position focale de l'unité de caméra (11) pour le même facteur de grossissement.

10. Microscope électronique (10) selon l'une au moins des revendications précédentes,
dans lequel
le dispositif de calcul (28) est adapté à subdiviser en plusieurs zones d'image les ensembles de données d'image primaire générés pour lesdites plusieurs valeurs focales et à les évaluer quant à un niveau de netteté respectif à l'intérieur des zones d'image, et à déterminer l'ensemble de données d'image secondaire du fait que pour chacune desdites plusieurs zones d'image, les données de cet ensemble de données d'image primaire sont sélectionnées qui présente le plus haut niveau de netteté.

11. Microscope électronique (10) selon la revendication 10,
dans lequel
le dispositif de calcul (28) est adapté à évaluer les ensembles de données d'image primaire quant au niveau de netteté du fait que pour la zone d'image respective, à la base de l'ensemble respectif de données d'image primaire, au moins une différence ou au moins un quotient est formé(e) entre les données d'image d'au moins deux éléments d'image voisins.

12. Microscope électronique (10) selon l'une au moins des revendications précédentes,
dans lequel
le dispositif de commande (24), le dispositif de calcul (28) et le viseur électronique (30) sont synchronisés les uns par rapport aux autres et sont adaptés
- à générer les ensembles de données d'image primaire pour lesdites plusieurs valeurs focales d'un cycle,
- à déterminer l'ensemble respectif de données d'image secondaire, et
- à afficher l'image respective
à une fréquence qui est d'au moins 25 Hz.

13. Microscope électronique (10) selon l'une au moins des revendications précédentes,
dans lequel
le viseur électronique (30) comprend deux dispositifs d'affichage électroniques (31) ou un dispositif d'affichage électronique (31) subdivisé en deux zones d'affichage, le viseur électronique (30) étant adapté à afficher l'image respective du canal de droite et du canal de gauche sur les deux dispositifs d'affichage (31) ou sur les deux zones d'affichage.

14. Microscope électronique (10) selon l'une au moins des revendications précédentes,
dans lequel
le microscope électronique (10) est susceptible de fonctionner au choix dans un premier mode de fonctionnement de profondeur de champ étendue ou dans un second mode de fonctionnement de résolution temporelle accrue ;
dans le premier mode de fonctionnement, les ensembles de données d'image secondaire sont déterminés à partir des ensembles de données d'image primaire générés pour lesdites plusieurs valeurs focales et ils sont affichés sur le viseur électronique (30) ; et
dans le second mode de fonctionnement, le dispositif de commande (24) est adapté à piloter le capteur d'image (16) en vue de générer des ensembles de données d'image primaire sans piloter le dispositif de réglage (22) en vue d'une modification répétée cycliquement de la position focale de l'unité de caméra entre lesdites plusieurs valeurs focales, et le viseur électronique (30) est adapté à afficher une image correspondant à l'ensemble respectif de données d'image primaire.
